Europäisches Patentamt

(19) European Patent Office

Office européen des brevets

(11) Publication number: 0 129 371
A2

(12) EUROPEAN PATENT APPLICATION

(21) Application number: 84303840.7

(22) Date of filing: 06.06.84

(51) Int. Cl.³: C 07 D 501/36
A 61 K 31/545

(30) Priority: 17.06.83 GB 8316589

(43) Date of publication of application:
27.12.84 Bulletin 84/52

(84) Designated Contracting States:
AT BE CH DE FR GB IT LI LU NL SE

(71) Applicant: IMPERIAL CHEMICAL INDUSTRIES PLC
Imperial Chemical House Millbank
London SW1P 3JF(GB)

(72) Inventor: Loftus, Frank
Oak dene, Langley Road Langley
Macclesfield Cheshire SK11 0DG(GB)

(74) Representative: Brown, Ivor James Stewart et al,
Imperial Chemical Industries PLC Legal Department:
Patents PO Box 6
Welwyn Garden City Herts, AL7 1HD(GB)

(54) Crystalline base-addition salts of an antibacterial cephalosporin derivative.

(57) A crystalline base-addition salt of 7-(imidazol -2-yl) amino-3-(1H-1,2,3-triazol-4-yl)- thiomethyleph -3-em-4 carboxylic acid in which the base is selected from N-methylpiperidine, N-ethylpiperidine, triethylamine or N-ethylmorpholine. A manufacturing process and pharmaceutical compositions are also described.

TITLE MODIFIED
see front page

0129371

PH. 32760|EP

TITLE:   CRYSTALLINE SALTS

This invention relates to crystalline salts of an antibacterial cephalosporin derivative.

In European Patent Publication 31708, Example 19, there is described the preparation of 7-(imidazol-2-yl)amino-3-(1H-1,2,3-triazol-4-yl)thiomethylceph-3-em-4-carboxylic acid.  This compound, both in the form of its common acid-addition salts and in the form of free acid-free base (zwitterion), is non-crystalline. However, it has now been discovered that certain base-addition salts can be obtained in crystalline form.

According to the invention there is provided a crystalline base-addition salt of 7-(imidazol-2-yl)-amino-3-(1H-1,2,3-triazol-4-yl)thiomethylceph-3-em-4-carboxylic acid in which the base is selected from N-methylpiperidine, N-ethylpiperidine, triethylamine or N-ethylmorpholine.

The crystalline salt of the invention is particularly useful for aiding the final purification of the cephalosporin derivative.  Thus in one easy step the purity of the cephalosporin derivative can be raised from about 90% or less to nearly 100%;  avoiding the need for extensive chromatography.   In addition the cephalosporin derivative exhibits improved stability in the crystalline salt form.

A particularly useful salt is that formed with triethylamine.

According to a further feature of the invention there is a provided a process for the manufacture of the crystalline salts described above. The process is characterised by addition of N-methylpiperidine, N-ethylpiperidine, triethylamine or N-ethylmorpholine to a suspension, partial solution or solution of 7-(imidazol-2-yl)amino-3-(1H-1,2,3-triazol-4-yl)thio-methylceph-3-em-4-carboxylic acid, either in the free acid-free base (zwitterionic) form, or in the form of an acid-addition salt, in a diluent or solvent. A particular diluent or solvent is acetonitrile, dimethylformamide, methanol or water, or a mixture of any two or three of these. The preferred diluent or solvent is dimethylformamide. The cephalosporin derivative and the base are preferably both in solution before crystallisation occurs. Alternatively it may be necessary to filter the mixture before crystallisation occurs, and it may also be necessary to concentrate the solution to induce crystallisation. Crystallisation of the salt generally takes place at ambient temperature, though it may be necessary to cool the mixture, for example to 0-10°C, preferably 4-5°, to induce crystallisation and/or improve the yield of crystalline product.

The crystalline salt of the invention may be used in the form of a pharmaceutical composition, in association with a pharmaceutically-acceptable diluent or carrier.

The pharmaceutical composition of the invention may, for example, be in a form suitable for oral, rectal or parenteral administration, for which purposes it may be formulated by means known to the art into the form of, for example, tablets, capsules, aqueous or oily solutions or suspensions, emulsions, dispersible powders, suppositories and sterile injectable aqueous or oily solutions or suspensions.

In addition to the crystalline salt of the invention the pharmaceutical composition of the invention may also contain, or be co-administered with, one or more known drugs selected from other clinically useful antibacterial agents (for example other $\beta$ - lactams or aminoglycosides), inhibitors of $\beta$-lactamase (for example clavulanic acid), renal tubular blocking agents (e.g. probenicid) and inhibitors of metabolising enzymes (for example inhibitors of peptidases, for example Z-2-acylamino-3-substituted propenoates).

A preferred pharmaceutical composition of the invention is one suitable for intravenous, subcutaneous or intramuscular injection, for example a sterile injectable containing between 1 and 5% w/w of the crystalline salt, or one suitable for oral administration in unit dosage form, for example a tablet or capsule which contains between 50mg. and 200mg. of the crystalline salt.

The pharmaceutical composition of the invention will normally be administered to man in order to combat infections caused by bacteria, in the same general manner as that employed for cephalothin, cefoxitin, cephradine and other known clinically used cephalosporin derivatives, due allowance being made in terms of dose levels for the potency of the crystalline salt of the present invention relative to the known clinically used cephalosporins. Thus each patient will receive a daily intravenous, subcutaneous or intramuscular dose of 50mg. to 500mg. and preferably 100mg. to 200mg., of the crystalline salt, the composition being administered 1 to 4 times per day. The intravenous, subcutaneous and intramuscular dose will be given by means of a bolus injection. Alternatively the intravenous dose may be given by continuous infusion over a period of time. Alternatively each patient will receive a daily oral dose which is approximately equivalent to the daily parenteral dose. Thus a preferred daily oral dose is 100mg. to 1g., and preferably 200mg. to 400mg., of the crystalline salt, the composition being administered 1 to 4 times, and preferably twice, per day.   The preferred route of administration is oral.

The invention is illustrated, but not limited by the following Examples:-

Example 1

To a suspension of 7-(imidazol-2-yl)amino-3-(1H-1,2,3-triazol-4-yl)thiomethylceph-3-em-4-carboxylic acid (European Patent Publication 31708, Example 19; 0.416 g.; 91% strength) in acetonitrile (10 ml.) and methanol (10 ml.) at ambient temperature was added N-ethylpiperidine (0.137 ml.), to give a partial solution.   The suspended solid was removed by filtration, and the filtrate evaporated under reduced pressure to a volume of about 5 ml.   The solution was stored at 4°C for 17 hours.   The crystalline solid which had formed was collected, washed with methanol and then acetonitrile and dried under reduced pressure to give the crystalline N-ethylpiperidine salt (40%) as fine colourless non-hygroscopic needles, m.p. 180°C. (decomp.), purity estimated by HPLC 99.4%.

Example 2

The process described in Example 1 was repeated, using triethylamine (0.14 ml.) in place of N-ethylpiperidine.   There was thus obtained the corresponding triethylamine salt (71%), as colourless, non-hygroscopic needles, m.p. 165°C (decomp.), purity estimated by HPLC 99.1%.   This salt can be recrystallised from water.

Example 3

To a solution of 7-(imidazol-2-yl)amino-3-(1H-1,2,3-triazol-4-yl)thiomethylceph-3-em-4-carboxylic acid (0.595 g., 84.4% strength) in DMF (5 ml.) was added triethylamine (0.2 ml.) and the resulting clear solution was stirred at ambient temperature to allow crystallisation to occur. After 30 minutes the resulting crystals were collected by filtration, washed with DMF (1.5 ml.), then acetonitrile, and dried at room temperature under reduced pressure to give the crystalline triethylamine salt (91%) as fine colourless non-hygroscopic needles, m.p. 165° (decomp), purity estimated by HPLC 97.7% with 0.99% water (Karl Fischer).

Example 4

The process described in Example 3 was repeated, using N-methylpiperidine in place of triethylamine. There was thus obtained the corresponding crystalline N-methylpiperidine salt (49%) as fine colourless non-hygroscopic needles, m.p. 185°C (decomp.), purity estimated by HPLC 100% with 0.6% water (Karl Fischer).

Example 5

7-(Imidazol-2-yl)amino-3-(1H-1,2,3-triazol-4-yl)thiomethylceph-3-em-4-carboxylic acid (5g.; 95.9% strength) was added gradually to a stirred solution of N-ethylmorpholine (2.3ml.) in water (7.5ml.) making sure that each aliquot had dissolved before the next was added. The resulting clear yellow solution was scratched to initiate crystallisation and the mixture was stored at 4°C. for 1 hour to permit crystallisation to take place. Methanol (42.5ml.) was then added in 5ml. aliquots over 2 hours with stirring, the temperature of the suspension being maintained at 4°C. The resulting crystalline suspension was stirred at 4°C. for a further 30 minutes and the crystals were collected by filtration, washed with methanol (5ml.) followed by acetonitrile (2 x 5ml.) and dried over $P_2O_5$ under reduced pressure to give the crystalline N-ethylmorpholine salt (53.6%) as fine colourless non-hygroscopic needles, m.p. 195°C (decomp.), purity estimated by HPLC 98.5% with 1.78% water (Karl Fischer).

Claims

1.      A crystalline base-addition salt of 7-
(imidazol —2-yl)amino-3-(1H-1,2,3-triazol-4-yl)-
thiomethylceph-3-em-4-carboxylic acid in which the base
is selected from N-methylpiperidine, N-ethylpiperidine,
triethylamine and N-ethylmorpholine.

2.      A crystalline salt as claimed in claim 1 in
which the base is triethylamine.

3.      A process for the manufacture of a crystalline
salt as claimed in claim 1 characterised by addition of
N-methylpiperidine, N-ethylpiperidine, triethylamine or
N-ethylmorpholine to a suspension, partial solution or
solution of 7-(imidazol-2-yl)amino-3-(1H-1,2,3-triazol-
4-yl)thiomethylceph-3-em-4-carboxylic acid, either on
the free acid-free base (zwitteronic) form, or in the
form of an acid-addition salt, in a diluent or solvent.

4.      A pharmaceutical composition which comprises a
crystalline salt as claimed in claim 1 in association
with a pharmaceutically-acceptable diluent or carrier.

BS32670
SC13
24 May 84

## Austrian Claim

1.      A process for the manufacture of a crystalline base-addition salt of 7-(imidazol-2-yl)amino-3-(1H-1,2,3-triazol-4-yl)-thiomethylceph-3-em-4-carboxylic acid in which the base is selected from N-methylpiperidine, N-ethylpiperidine, triethylamine and N-ethylmorpholine characterised by addition of N-methylpiperidine, N-ethylpiperidine, triethylamine or N-ethylmorpholine to a suspension, partial solution or solution of 7-(imidazol-2-yl)amino-3-(1H-1,2,3-triazol-4-yl)thiomethylceph-3-em-4-carboxylic acid, either in the free acid-free base (zwitteronic) form, or in the form of an acid-addition salt, in a diluent or solvent.

BS32670EP(Austria)

SC13

24 May 84